# EUROPÄISCHE PATENTSCHRIFT

(11) **EP 3 303 438 B1**
(45) Veröffentlichungstag und Bekanntmachung des Hinweises auf die Patenterteilung: **21.10.2020**
(21) Anmeldenummer: 16726316.9
(22) Anmeldetag: 01.06.2016
(51) Int. Cl.: C08G 18/67, C08G 18/79, C08G 18/28, C04B 103/32, A61F 13/00, C04B 24/28, C04B 26/16, C08G 18/48, C08G 18/75, C04B 103/00, C04B 111/00

(54) **VINYL-TERMINIERTE PREPOLYMERE MIT NIEDRIGER VISKOSITÄT UND GUTER WASSERLÖSLICHKEIT**
VINYL-TERMINATED PREPOLYMERS WITH LOW VISCOSITY AND GOOD WATER SOLUBILITY
PRÉ-POLYMÈRE À TERMINAISON VINYLE AYANT UNE FAIBLE VISCOSITÉ ET UNE BONNE SOLUBILITÉ DANS L'EAU

(30) Priorität: 03.06.2015 EP 15170539
(43) Veröffentlichungstag der Anmeldung: 11.04.2018
(73) Patentinhaber: Sika Technology AG, 6340 Baar (CH)
(72) Erfinder: HAUFE, Markus, 8048 Zürich (CH); HUG, Max, 8135 Langnau a. Albis (CH)
(74) Vertreter: Sika Patent Attorneys
(86) Internationale Anmeldenummer: PCT/EP2016/062374
(87) Internationale Veröffentlichungsnummer: WO 2016/193302

(56) Entgegenhaltungen:
- EP-A1- 2 581 396
- WO-A1-2007/063027
- DATABASE WPI Week 200416 Thomson Scientific, London, GB; AN 2004-159412 XP002431578, -& JP 2003 201331 A (NIPPON SYNTHETIC CHEM IND CO) 18. Juli 2003 (2003-07-18)

## Beschreibung

### Technisches Gebiet

Die Erfindung betrifft die Verwendung von vinylterminierte Prepolymere auf Basis von Polyethern als Injektionsmittel oder Bestandteil eines Injektionsmittel zur Abdichtung von Bauwerken, Tunneln oder Minen, wobei die vinylterminierte Prepolymere durch die Umsetzung von Polyethern mit gegenüber Isocyanat-reaktiven funktionellen Gruppen mit Isocyanaten einer mittleren Isocyanatfunktionalität im Bereich von 2,4 bis 3,5 und Vinylverbindungen, die ebenfalls eine gegenüber Isocyanat reaktive funktionelle Gruppe aufweisen, hergestellt werden, wobei die Polyether zu den Vinylverbindungen, die Summe der Molmengen der Polyether und Vinylverbindungen zu Isocyanatgruppen und der Anteil der Polyether in den vinylterminierten Prepolymere in bestimmten Verhältnissen vorliegen. Darüber hinaus betrifft die vorliegende Erfindung Verfahren zur Herstellung eines Injektionsmittels umfassend die Herstellung eines vinylterminierten Prepolymers sowie deren Verwendung zur Herstellung von Superabsorbern und Betonverflüssigern.

### Stand der Technik

Konventionelle radikalisch härtende Systeme sind in der Regel aus Monomeren wie Allyl-, Vinyl-, Methacryl- und Acryl-Verbindungen aufgebaut. Bei der Herstellung von hydrophilen Systemen kommen Monomere zum Einsatz, die hydrophile Gruppen, beispielswese in Form von Hydroxy- oder Ammoniumgruppen, aufweisen. Werden hydrophile Polymere direkt durch radikalische Polymerisation, d.h. nicht über den Umweg einer Polymerisation unpolarer Estermonomere und deren anschließende Verseifung zu Alkoholen, hergestellt, kommen häufig Monomere wie Hydroxyalkyl (meth)acrylate, (Meth)acrylsäure oder Polyalkylenglykole zum Einsatz, deren terminale Hydroxyfunktionen mit (Meth)acrylaten modifiziert sind. Ein Beispiel solcher Polyalkylenglykol-(meth)acrylate sind insbesondere Polyethylenglykol-Dimethacrylate, die nach einer Polymerisation günstige Eigenschaften als Dichtmittel gegenüber Wasser aufweisen. Ein Beispiel für die Verwendung von Polyalkylenglykoldi-(meth)acrylaten findet sich in der EP 2 164 881 B1, in der Dichtmittel auf Basis von Polyethylenglykol-dimethacrylaten mit einem gewichtsmittleren Molekulargewicht von mehr als 5000 g/Mol vorgeschlagen werden. Im Vergleich zu entsprechenden Dichtmitteln auf Basis von Polyethylenglykolen mit geringerem Molekulargewicht sollen sich die beanspruchten Dichtmittel über eine verbesserte Quellbarkeit und eine erhöhte Bruchdehnung auszeichnen.

Bei Injektionsapplikationen sind möglichst niedrige Viskositäten unabdingbar, da die Monomere über enge Zugänge an den gewünschten Applikationsort gebracht werden müssen, und einen zu füllenden Hohlraum auch möglichst vollständig ausfüllen sollen. Dies gilt insbesondere für Rissabdichtungsanwendungen, bei denen sich das eingebrachte Material in sehr schmalen Spalten verteilen muss. Während Monomere wie Hydroxyalkyl (meth)acrylate oder (Meth)acrylsäure entsprechend niedrige Viskositäten aufweisen, nimmt die Viskosität von Polyethylenglykol-Di(meth)acrylatverbindungen mit Molekularmassen von > 500 Mn stark zu, was deren Eignung für Injektionsapplikationen beeinträchtigt. Trotz dieses Nachteils werden Polyethylenglykol-Di(meth)acrylatverbindungen auch in Injektionsapplikationen eingesetzt, da diese Monomere zu quellfähigen Polymeren umgesetzt werden können.

Im Stand der Technik werden Polyethylenglykol-Di(meth)acrylatverbindungen in der Regel durch Umesterung leicht zugänglicher (Meth)acrylatester oder durch direkte Veresterung von Polyethylenglykolen hergestellt. Die Herstellung über eine Umesterung, bei der eine Alkoholgruppe abgespalten wird, ist jedoch mit dem Nachteil verbunden, dass ein solches Reaktionsprodukt gemäß REACh registriert werden muss, was mit einem Zeitverlust und zusätzlichen Kosten verbunden ist. Um eine REACh Registrierung zu vermeiden, kann das Nebenprodukt zwar abgetrennt werden (bei der Umesterung von Methylmethacrylat mit Polyethylenglycol entsteht z.B. Methanol als Nebenprodukt, dass im Unterdruck entfernt werden kann), auch dies ist jedoch mit einem zusätzlichen Verarbeitungsschritt und damit mit höheren Kosten verbunden.

Bei einer direkten Veresterung eines Polyethylenglykols mit einem Vinylanhydrid kommt es zur Entstehung von Nebenprodukten, da beispielsweise bei Verwendung eines Säureanhydrids ein Säureanion als Abgangsgruppe freigesetzt wird. Derartige Nebenprodukte sind zum einen gemäß REACh registrierpflichtig, zum anderen müssen sie aber auch aus dem erhaltenen Produkt abgetrennt und entweder aufgearbeitet oder entsorgt werden. Eine Abtrennung lässt sich bei Verwendung eines cyclischen Anhydrids (z.B. Maleinsäureanhydrid) vermeiden. Nach der Umsetzung verbleibt aber eine Säurefunktion im Produkt, so dass die resultierenden Prepolymere relativ polar sind. Dies wiederum kann in den aus den Prepolymeren hergestellten Polymeren unerwünscht sein.

Neben der Veresterung oder Umesterung können hydrophile Prepolymere mit terminalen Vinylfunktionen auch über NCO/OH- oder NCO/NH-Reaktionen herstellt werden. Denkbar ist beispielsweise eine Umsetzung von Polyetherpolyolen oder Polyethern mit terminalen Aminofunktionen mit einem Überschuss an Diisocyanat, und die anschließende Umsetzung der Isocyanatmodifizierten Polyether mit einer ungesättigten Alkohol- oder Aminverbindung. Bei der Herstellung von vinylterminierten Prepolymeren über eine NCO/OH-Reaktion hat sich aber die relativ hohe Reaktivität der terminalen OH-Gruppen im Polyethylenglykol als problematisch erwiesen, die dazu führt, dass gezielte und stöchiometrische Umsetzungen nur mit großem Aufwand oder gar nicht möglich sind. In der Folge kommt es in einem gewissen Umfang immer zu einer Kettenverlängerung, was sich nachteilig auf die Viskosität der hergestellten Prepolymere auswirkt.

Ein im Stand der Technik beschriebener Ansatz zur Verringerung der Viskosität von Polyethylenglykoldiacrylaten besteht darin, an Stelle der reinen Polyethylenglykol-Diole Blockcopolymere auf Basis von Polyethylenglykol und Polypropylenglykol einzusetzen. Durch den Polypropylenglykolanteil kommt es zu einer Verminderung der Viskosität, die umso stärker ist, je höher der Polypropylenglykol-Anteil im Copolymer ist. Nachteilig an diesem Ansatz ist jedoch, dass mit zunehmendem Anteil an Polypropylenglykol die Wasserlöslichkeit der resultierenden Prepolymere negativ beeinflusst wird. Ein weiteres Problem besteht darin, dass solche Prepolymere als Tenside wirken, wodurch es bei der Endanwendung zu unerwünschtem Schäumen kommen kann. Darüber hinaus sind auch die erwähnten Polyethylenglykol/Polypropylenglykol-Blockcopolymere oftmals so reaktiv, dass bei der Umsetzung mit Isocyanaten relativ hochviskose Prepolymere gebildet werden. Ein weiterer Nachteil besteht darin, dass sich der Polypropylenanteil negativ auf die Quellfähigkeit der auspolymerisierten Prepolymere auswirkt.

Die EP 2 581 396 beschreibt ein Verfahren zur Herstellung von niedrigviskosen, wasserverdünnbaren Urethan(meth)acrylaten, die durch Umsetzung von oligomeren Polyisocyanaten, Polyoxyalkylenmono-olen, Hydroxyalkyl(meth)acrylaten und Polyoxyalkylenpolyolen auf Basis von Startermolekülen mit mindestens drei Hydroxyfunktionalitäten, die teilweise durch Vernetzung mit (Meth)acyrlsäure so umgesetzt sind, dass im Mittel 0,2 bis 1,5 Hydroxyfunktionalitäten verbleiben, erhältlich sind. Die beschriebenen wasserverdünnbaren Urethan(meth)acrylate sollen insbesondere als Beschichtungsmittel verwendbar sein, da ihre Viskosität durch Zugabe von Wasser variabel eingestellt werden kann.

Die JP 2003-201331 A beschreibt Strahlen-härtbare Beschichtungszusammensetzungen, die Polyisocyanatderivate aus Polyisocyanatverbindungen, spezifischen Polyoxyalkylenglykol-Derivaten und Hydroxylgruppen enthaltenden (Meth)acryaten beinhalten. Über die Isocyanatreaktion sind die Polyoxyalkylenglykol-Derivate und Hydroxylgruppen enthaltenden (Meth)acryaten kovalent an die Polyisocyanate gebunden.

Die WO 2007/063027 beschreibt Strahlen-härtbare wasseremulgierbare Polyisocyanate auf Basis von Umsetzungsprodukten von organischen Di- oder Polyisocyanaten, Verbindungen mit einer gegenüber Isocyanat-reaktiven und einer radikalisch polymerisierbaren funktionellen Gruppe, sowie einer Verbindung mit einer gegenüber Isocyanat-reaktiven Gruppe und einer gesättigten dispergieraktiven Gruppe. Mit solchen Polyisocyanten sollen sich Oberflächenbeschichtungen mit verbesserter Härte, Kratzfestigkeit, Chemikalienbeständigkeit, Haftung und Elastizität herstellen lassen.

Vor diesem Hintergrund besteht ein Bedarf an vinylterminierten Prepolymeren auf Basis von Polyethern, die sich sowohl durch eine möglichst geringe Viskosität als auch eine gute Wasserlöslichkeit auszeichnen. Die vorliegende Erfindung befasst sich mit diesem Problem.

### Darstellung der Erfindung

Aufgabe der vorliegenden Erfindung ist es demzufolge, die aus dem vorstehend geschilderten Stand der Technik bekannten vinylterminierten Prepolymere zu verbessern und insbesondere Prepolymere vorzuschlagen, die als Injektionsmittel oder Bestandteil eines Injektionsmittel zur Abdichtung von Bauwerken, Tunneln oder Minen verwendet geeignet sind und die sich im Vergleich zu den bekannten Systemen durch eine niedrige Viskosität und eine gute Wasserlöslichkeit auszeichnen. Die Prepolymere sollten darüber hinaus in wässriger Lösung über einen längeren Zeitraum stabil sein, ohne dass es zu einer Verfestigung oder Polymerisation der Materialien kommt. Schließlich sollten die Prepolymere über einfache Umsetzungen herstellbar sein, bei denen möglichst keine Nebenprodukte anfallen, die abgetrennt oder entsorgt werden müssen. Trotz dieser Vorgaben sollten die Prepolymere leicht modifizierbar sein, um so eine einfache Abstimmung der Eigenschaften auf den Anwendungszweck zu ermöglichen. Entsprechend den bekannten vinylterminierten Prepolymeren sollten die weiterentwickelten Prepolymere radikalisch polymerisierbar sein.

Erfindungsgemäß wird dies durch die Verwendung eines vinylterminiertes Prepolymer auf Basis von Polyethern als Injektionsmittel oder Bestandteil eines Injektionsmittel zur Abdichtung von Bauwerken, Tunneln oder Minen nach Anspruch 1 erreicht. Die vorliegende Erfindung befasst sich zudem mit Verfahren zur Herstellung eines Injektionmittels umfassend die Herstellung derartiger vinylterminierter Prepolymere, deren Verwendung zur Herstellung von Superabsorbern sowie deren Verwendung zur Herstellung von Betonverflüssigern die durch Polymerisation des vorstehend angegebenen vinylterminierten Prepolymers, gegebenenfalls unter Zusatz weiterer Vinylmonomere, erhältlich sind.

Gemäß einem ersten Aspekt betrifft die vorliegende Erfindung die Verwendung eines vinylterminierten Prepolymers auf Basis von Polyethern zur Abdichtung von Bauwerken, Tunneln oder Minen, das durch Umsetzung
i) eines Polyethers mit genau einer gegenüber Isocyanaten reaktiven funktionellen Gruppe mit
ii) mindestens einem Polyisocyanat, das eine mittlere Isocyanatfunktionalität im Bereich von 2,4 bis 3,5 aufweist, und
iii) mindestens einer Vinylverbindung, die genau eine gegenüber Isocyanaten reaktive funktionelle Gruppen aufweist, erhältlich ist,
wobei das Molverhältnis von Polyether i) zu Vinylverbindung iii) im Bereich von 3:1 bis 1:3 liegt und das Verhältnis der Summe der Molmengen von Polyether und Vinylverbindung zu Isocyanatgruppen ii) im Bereich von 1,5:1 bis 1:1,5 liegt und wobei der Anteil des Polyethers im vinylterminierten Prepolymer mindestens 30 Gew.-% und bevorzugt mindestens 33 Gew.-%, bezogen auf das Gesamtgewicht des Prepolymers, ausmacht.

Bei dem "mindestens einen Polyisocyanat", das eine mittlere Isocyanatfunktionalität im Bereich von 2,4 bis 3,5 aufweist, bezeichnet die mittlere Isocyanatfunktionalität die mittlere molare Isocyanatfunktionalität der Bestandteile des mindestens einen Isocyanats. Besteht das mindestens eine Isocyanat beispielsweise aus einer 1:1-Mischung aus einem Diisocyanat und einem Triisocyanat, so ergibt sich eine mittlere Isocyanatfunktionalität von 0,5 x 2 + 0,5 x 3 = 2,5. Besteht das mindestens eine Isocyanat ausschließlich aus Triisocyanaten, ergibt sich eine mittlere Isocyanatfunktionalität von 1 x 3 = 3.0. Handelt es sich bei dem mindestens einen Polyisocyanat um ein Polyisocyanat, so ist die mittlere molare Isocyanatfunktionalität eine ganze Zahl, d.h. im vorliegenden Fall 3,0.

Bei den "gegenüber Isocyanat-reaktiven funktionellen Gruppen" handelt es sich bevorzugt um Hydroxy-, Amino- oder Thiolgruppen, von denen Amino-und Hydroxylgruppen besonders bevorzugt und Hydroxylgruppen am meisten bevorzugt sind.

Mit "einer" gegenüber Isocyanaten reaktiven funktionellen Gruppe ist gemeint, dass besagte Polyether bzw. die mindestens eine Vinylverbindung genau eine (d.h. im Sinne einer Anzahl) gegenüber Isocyanaten reaktive funktionelle Gruppe aufweisen sollen. Umfasst die mindestens eine Vinylverbindung mehr als eine Vinylverbindung, so ist die Angabe "mit einer gegenüber Isocyanaten reaktiven funktionellen Gruppe", so zu verstehen, dass jede der Vinylverbindungen eine gegenüber Isocyanaten reaktive funktionelle Gruppe aufweist.

Von der Bezeichnung "(Meth)acrylat" sind im Folgenden immer sowohl Methacrylate als auch Acrylate erfasst.

Das Molekulargewicht bezeichnet im Rahmen der vorliegenden Erfindung, sofern nicht anders angegeben, das gewichtsmittlere Molekulargewicht Mw, dass zweckmäßig durch GPC mit Hilfe geeigneter Standards (z.B. Polystyrol) zu bestimmen ist.

Bei dem Polyether, der die Grundlage des Polyethers mit einer gegenüber Isocyanaten reaktiven funktionellen Gruppe bildet, handelt es sich bevorzugt um ein, zweckmäßig lineares, Polyalkylenglykol, bei dem mit Ausnahme einer OH-Gruppe alle weiteren vormals im Polyalkylenglykol vorhandenen gegenüber Isocyanaten reaktiven funktionellen Gruppen mit einer Capping-Gruppe versehen und dadurch deaktiviert wurden. Zum Beispiel kann eine OH-Gruppe zu einem Ether modifiziert werden, der gegenüber Isocyanaten unreaktiv ist. Analog kann eine Aminogruppe zu einem Amid modifiziert werden. Für die Capping-Gruppe entscheidend ist jedoch allein, dass sie keine gegenüber Isocyanaten reaktiven funktionellen Gruppen und keine Vinylgruppen aufweist.

Bei dem Polyether handelt es sich bevorzugt um ein Polyethylenglykol, Polypropylenglykol oder ein Polyethylenglykol/Polypropylenglykol-co- oder Blockcopolymer. Für Polyethylenglykol/Polypropylenglykolcopolymere gilt es weiterhin als bevorzugt, wenn diese einen Polypropylenglykolanteil von 60 Mol% oder weniger und bevorzugt 50 Mol% oder weniger aufweisen. Besonders bevorzugt handelt es sich bei dem Polyether um ein Polyethylenglykol. Darüber hinaus ist es bevorzugt, wenn der Polyether bei Raumtemperatur flüssig ist.

Bei der Capping-Gruppe handelt es sich unabhängig davon bevorzugt um eine Alkoxygruppe und besonders bevorzugt eine Methoxygruppe.

Hinsichtlich des Molekulargewichts Mw unterliegt der Polyether mit einer gegenüber Isocyanaten reaktiven funktionellen Gruppe keinen wesentlichen Beschränkungen, solange der Polyether eine ausreichende Wasserlöslichkeit des resultierenden Prepolymers gewährleistet. Es gilt jedoch als zweckmäßig, wenn das Molekulargewicht Mw des Polyethers im Bereich von 200 bis 5000 g/mol, bevorzugt 300 bis 2000 g/mol und besonders bevorzugt 350 bis 1800 liegt.

Molekulargewichte von mehr als 5000 führen, wie vorstehend bereits angedeutet, zu vinylterminierten Prepolymeren mit erhöhten Viskositäten, während Polyether mit Molekulargewichten von weniger als 200 Prepolymere mit verminderter Wasserlöslichkeit bilden.

Die Vinylverbindung, die eine gegenüber Isocyanaten reaktive funktionelle Gruppe aufweist, kann, wie vorstehend beschrieben, eine Vinylverbindung sein, die Hydroxy- oder Aminogruppen aufweist. Bevorzugt ist die mindestens eine Vinylverbindung demzufolge ausgewählt aus Alkoholen und Aminen. Geeignete Alkohole sind beispielsweise Ester von α,β-ungesättigten Carbonsäuren mit einem Diol, z. B. 2-Hydroxyethyl(meth)acrylat (HEMA), 3-und 2-Hydroxypropyl(meth)acrylat (HPMA), 1,3-Dihydroxypropyl-2-(meth)acrylat, 2,3-Dihydroxypropyl-1-(meth)acrylat, 2-, 3- oder 4-Hydroxybutyl(meth)acrylat und Isomere von Hydroxyhexyl(meth)acrylaten. Weiterhin geeignet sind Monoester von α,β-ungesättigten Carbonsäuren, z.B. in Form von (Meth)acrylsäure, mit Polyethylen- oder Polypropylenglycol, 2-Hydroxy-3-phenoxypropyl acrylat, Addukte von α,β-ungesättigten Carbonsäuren mit Caprolacton, Amide von α,β-ungesättigten Carbonsäuren mit Aminoalkoholen, wie N-Hydroxymethyl acrylamid oder N-Hydroxyethyl acrylamid sowie Additionsprodukte davon mit Ethylen- oder Propylenoxid, Alkanolvinylether, wie z. B. 2-Hydroxyethylvinylether, 4-Vinylbenzylalkohol, oder Allylalkohol sowie Additionsprodukte von Allylalkohol und Ethylen- oder Propylenoxid oder p-Methylolstyrol. Vorzugsweise wird das hydroxyfunktionelle Vinylmonomer aus der Klasse der Hydroxyalkyl(meth)acrylate ausgewählt. Besonders bevorzugte Alkohole sind 2-Hydroxyethylmethacrylat, 3- und 2-Hydroxypropyl(meth)acrylat und Allylalkohol. Die Bezeichnung 2-Hydroxypropyl(meth)acrylat umfasst dabei beide möglichen Konstitutionsisomere, sowie Gemische davon.
Geeignete Amine sind primäre Amine auf Basis von Amiden der (Meth)acrylsäure mit aliphatischen Diaminen wie Ethylendiamin, 1,3-Propylendiamin sowie analogen α,ω-Diaminen mit 4 bis 10 Kohlenstoffatomen und Allylamin. Geeignete sekundäre Amine, sind Ester der (Meth)acrylsäure mit Aminoalkoholen wie 2-(tert-Butylamino)ethyl (meth)acrylat, und Amide der (Meth)acrylsäure mit Aminen, die eine primäre und eine sekundäre Aminogruppe enthalten, z.B. Alkylaminoalkyl(meth)acrylate.

Das Prepolymer enthält neben den vorstehend erwähnten Amin- und Alkohol-Bestandteilen bevorzugt, keine wesentlichen Anteile an weiteren Alkohol-Bestandteilen, die über die Isocyanatreaktion in das Prepoylmer einbezogen werden. So enthält das Prepolymer z.B. zweckmäßig weniger als 10 Gew.-%, insbesondere weniger als 5 Gew.-% und besonders bevorzugt weniger als 1 Gew.-% an Reaktionsprodukten aus Polyoxyalkylenpolyolen auf Basis von Startermolekülen mit drei oder mehr Hydroxyfunktionalitäten, in denen die Hydroxyfunktionalitäten ganz oder teilweise mit Methacrylsäure umgesetzt wurden. Besonders bevorzugt ist es, wenn das Prepolymer im Wesentlichen aus den vorgenannten Alkohol- und/oder Aminbestandteilen, sowie den im folgenden erwähnten Polyisocyanaten besteht (d.h. zu mindestens 95 Gew.-% und bevorzugt mindestens 98 Gew.-%); meist bevorzugt besteht das Prepolymer aus diesen Bestandteilen.

Das mindestens eine Polyisocyanat umfasst bevorzugt ein aliphatisches Triisocyanat, das auch durch Trimerisierung eines aliphatischen Diisocyanats gebildet werden kann. In solchen Trimeren können die Diisocyanateinheiten über eine Cyanursäurefunktion verknüpft sein, in die jeweils eine der Isocyanatfunktionen des Diisocyanats einbezogen ist. Besonders geeignete Ausgangsprodukte für solche Trimere sind beispielsweise Alkylendiisocyanate. Besonders bevorzugt umfasst das Polyisocyanat ein Hexamethylendiisocyanat-Trimer.

Im Rahmen der vorliegenden Erfindung hat es sich weiterhin als zweckmäßig herausgestellt, wenn in das Prepolymer neben einem Triisocyanat zusätzlich ein Diisocyanat einbezogen wird. Bei diesem Diisocyanat handelt es sich vorzugsweise um ein aliphatisches Diisocyanat und besonders bevorzugt um Isophorondiisocyanat. Durch den Zusatz eines solchen Diisocyanates in geringen Mengen wird eine Verfestigung (Selbst-Thixotropierung) des Prepolymers bei längerem Stehenlassen unterbunden, so dass das hergestellte Prepolymer auch nach längerer Lagerung noch problemlos verarbeitet werden kann.

Wie vorstehend angegeben weist das Polyisocyanat eine mittlere Isocyanatfunktionalität im Bereich von 2,4 bis 3,5 auf. Als bevorzugte Bereiche für die mittlere Isocyanatfunktionalität kann ein Bereich von 2,5 bis 3,1 und insbesondere von 2,7 bis 3,0 angegeben werden.

Das Molverhältnis des Polyethers i) zur Vinylverbindung iii) (bzw. der Gesamtheit der Vinylverbindungen) liegt bevorzugt im Bereich von 2:1 bis 1:2 was zur Folge hat, dass bei einer mittleren Isocyanatfunktionalität von drei im Mittel mindestens 1 Vinylverbindung und mindestens ein Polyetherrest im Prepolymer enthalten sind. Durch einen höheren Anteil von Polyether wird bei einer Polymerisation eine geringere Vernetzung bei einem höheren Anteil von Polyethern im Produkt realisiert, was eine bessere Quellfähigkeit des Produkts bei Kontakt mit Wasser zur Folge hat. Für Anwendungen, die eine höhere Quellfähigkeit erfordern, kann deshalb ein höherer Anteil von Polyethern im Prepolymer eingestellt werden. Ein höherer Anteil an Vinylverbindung iii) führt demgegenüber zu einer höheren Vernetzung und in der Folge zu verbesserten mechanischen Eigenschaften. In einer Ausführungsform liegt das Molverhältnis des Polyethers i) zur Vinylverbindung iii) im Bereich von 1:1 bis 1:2 und bevorzugt im Bereich von 1:1,5 bis 1:2. In einer alternativen Ausführungsform liegt das Molverhältnis des Polyethers i) zur Vinylverbindung iii) im Bereich von 2:1 bis 1:1 und bevorzugt im Bereich von 1,5:1 bis 2:1.

Das Verhältnis der Summe der Molmengen von Polyether und Vinylverbindung zur Molmenge an Isocyanatgruppen liegt, wie vorstehend angegeben, im Bereich von 1,5:1 bis 1:1,5. Die Molmenge an Isocyanatgruppen bezeichnet hier die Molmenge an Isocyanatgruppen in der Mischung, und ist nicht zu verwechseln mit der Molmenge an Polyisocyanat. Als bevorzugt kann ein Bereich von 1,2:1 bis 1:1,2 und insbesondere 1,1:1 bis 1:1,1 angegeben werden, wobei ein leichter Überschuss an Isocyanat, wie ein Verhältnis von 1: 1,03 bis 1:1,1 bevorzugt ist um eventuell in den Ausgangsprodukten enthaltenes Wasser abzufangen.

Im Allgemeinen sollte der Anteil des Polyethers im vinylterminierten Prepolymer hoch genug sein, um die Wasserlöslichkeit des Prepolymer sowie eine günstige Quellfähigkeit zu gewährleiten. Als erfindunsgemässer Mindestanteil des Polyethers wird hier ein Anteil von mindestens 30 Gew.-%, bezogen auf das Gesamtgewicht des Prepolymers, insbesondere mindestens 33 Gew.-%, bevorzugt mindestens 35 Gew.-% und meist bevorzugt mindestens 40 Gew.-% angegeben. Andererseits sollte der Anteil des Polyethers, bezogen auf das Gesamtgewicht des Prepolymers, einen Wert von 80 Gew.-% nicht übersteigen, wobei Werte von bis zu 70 Gew.-% und insbesondere bis zu 60 Gew.-% als bevorzugt angegeben werden können.

Darüber hinaus ist es möglich, für die Umsetzung, durch die das vinylterminierte Prepolymer erhalten wird, eine aromatische oder aliphatische Verbindung einzubeziehen, die eine gegenüber Isocyanaten reaktive funktionelle Gruppe aufweist, aber weder als Polyether noch als Vinylverbindung iii) zu qualifizieren ist. So können beispielsweise durch Zusatz von Aminen oder von Alkoholen, wie Ethanol oder Propanol, gegebenenfalls nach der Reaktion noch vorhandene überschüssige Isocyanatfunktionalitäten in Urethane umgewandelt werden, um so ein völlig isocyanatfreies Produkt zu erhalten. Wird das Prepolymer unter Einbezug der vorbeschriebenen Verbindungen hergestellt, so ist bei der Berechnung der Summe der Molmengen von Polyether und Vinylverbindung auch die Molmenge dieser Verbindungen zu berücksichtigen.

Die Molmenge der zusätzlichen aromatischen oder aliphatischen Verbindungen sollte maximal so hoch sein wie die mittlere Isocyanatfunktionalität des Polyisocyanats minus 2. Dadurch wird sichergestellt, dass das erhaltene Prepolymer im Mittel eine Vinylfunktion und einen Polyetherrest enthält.

Ein weiterer Aspekt der vorliegenden Erfindung befasst sich mit Verfahren zur Herstellung eines Injektionsmittel umfassend die Herstellung der im Vorstehenden beschriebenen vinylterminierten Prepolymere. Ein solches Verfahren kann entweder sequentiell durchgeführt werden, indem zunächst eine der Komponenten mit einer gegenüber Isocyanaten reaktiven funktionellen Gruppe (d.h., der Polyether oder die Vinylverbindung) mit dem mindestens einen Polyisocyanat unter Erhalt eines Zwischenprodukts umgesetzt und anschließend die zweite Komponente mit einer gegenüber Isocyanaten reaktiven funktionellen Gruppe zugegeben wird, so dass diese Komponente mit den im Zwischenprodukt verbleibenden Isocyanatfunktionen reagieren kann. Es ist jedoch auch möglich, alle Bestandteile i) bis iii) in einer Eintopfreaktion umzusetzen, was aufgrund der leichteren Verfahrensführung und des geringeren Zeitaufwands bis zum Erhalt des Endprodukts bevorzugt ist.

In einer Ausführungsform handelt es sich bei dem erwähnten Verfahren zur Herstellung eines Injektionsmittel umfassend die Herstellung eines vinylterminierten Prepolymers, wie es im Vorstehenden beschrieben wurde, um ein Verfahren, umfassend
i) die Zugabe mindestens eines Polyisocyanats mit einer mittleren Isocyanatfunktionalität im Bereich von 2,4 bis 3,5 entweder zu einem Polyether mit genau einer gegenüber Isocyanaten reaktiven funktionellen Gruppe oder zu mindestens einer Vinylverbindung, die genau eine gegenüber Isocyanaten reaktive funktionelle Gruppen aufweist,
ii) das Abreagierenlassen der Isocyanatgruppen mit den gegenüber Isocyanaten reaktiven funktionellen Gruppen,
iii) abhängig von dem im Schritt i) vorgelegten Bestandteil, die Zugabe des anderen Bestandteils, zum Reaktionsprodukt aus Polyisocyanat und dem vorgelegten Bestandteil, und
iv) das Abreagierenlassen des Reaktionsprodukts aus Polyisocyanat und dem vorgelegten Bestandteil mit dem anderen Bestandteil, wobei
der "andere Bestandteil" die mindestens eine Vinylverbindung, die eine gegenüber Isocyanaten reaktive funktionelle Gruppe aufweist, ist wenn der im Schritt i) vorgelegte Bestandteil der Polyether mit einer gegenüber Isocyanaten reaktiven funktionellen Gruppe ist, und umgekehrt und die Zugabe eines Radikalstarters und ggf. weiterer Komponente.

In einer weiteren Ausführungsform handelt es sich bei dem erwähnten Verfahren zur Herstellung eines Injektionsmittel umfassend die Herstellung eines vinylterminierten Prepolymers, wie vorstehend beschrieben, um ein Verfahren, umfassend
i) die Zugabe von mindestens einem Polyisocyanat mit einer mittleren Isocyanatfunktionalität im Bereich von 2,4 bis 3,5 zu einer Mischung aus einem Polyether mit genau einer gegenüber Isocyanaten reaktiven funktionellen Gruppe und mindestens einer Vinylverbindung, die genau eine gegenüber Isocyanaten reaktive funktionelle Gruppen aufweist, und
ii) das Abreagierenlassen des Polyisocyanats mit dem Polyether und der Vinylverbindung.

Die vorgenannten Verfahren lassen sich in Bezug auf die erzielbare Umsetzungsgeschwindigkeit durch den Zusatz eines Katalysators optimieren, der die Reaktion der Isocyanatgruppen mit den gegenüber Isocyanaten reaktiven funktionellen Gruppen beschleunigt. Geeignete Katalysatoren sind in diesem Zusammenhang alle bekannten Katalysatoren für die Bildung von Urethanen, wie tertiäre Amine und Metallsalze, wie Zinnsalze, Titansalze und Bismuthsalze. Besonders geeignete Katalysatoren sind Dibutylzinnlaurat und Bismuthneodecanoat.

Hinsichtlich des Anteils des einzubeziehenden Katalysators unterliegt die vorliegende Erfindung keinen relevanten Beschränkungen, mit der Vorgabe, dass der Anteil des Katalysators auf ein Minimum des Erforderlichen beschränkt werden sollte. Besonders geeignete Anteile an Katalysator liegen im Bereich von 0,001 bis 1 Gew.-% und bevorzugt 0,005 bis 0,1 Gew.-%.

Darüber hinaus kann es sinnvoll sein, der Reaktionsmischung einen Radikalstabilisator zuzusetzen, um eine vorzeitige Polymerisierung des erhaltenen vinylterminierten Prepolymers zu unterbinden. Geeignete Stabilisatoren sind beispielsweise OH-TEMPO (4-hydroxy-2,2,6,6-tetramethylpiperidin-1-oxyl) oder Hydroquinon-monomethylether.

Ein weiterer Aspekt der vorliegenden Erfindung betrifft die Verwendung eines vinylterminierten Prepolymers, wie vorstehend beschrieben, als Injektionsmittel oder Bestandteil eines Injektionsmittels zur Gesteins- oder Bodenverfestigung. Erfindungsgemäss werden derartige Injektionsmittel zur Abdichtung von Bauwerken, Tunneln oder Minen eingesetzt. Im Rahmen dieser Verwendung wird das vinylterminierte Prepolymer mit einem Radikalstarter (Initiator) und gegebenenfalls weiteren Komponenten in einen Hohlraum eingespritzt und zweckmäßig dort polymerisiert.

Neben den beschriebenen vinylterminierten Prepolymeren kann das Injektionsmittel weitere Vinylmonomere, wie insbesondere (Meth)acrylate, enthalten.

Als Radikalstarter für die erwähnte Polymerisation können bekannte Initiatoren für Radikalreaktionen, wie beispielsweise Alkalimetallpersulfate, Ammoniumpersulfate und Wasserstoffperoxide oder Azo-bis-isobutyronitril (AIBN) oder organische Peroxide wie Dibenzoylperoxid eingesetzt werden. Wenn das Injektionsmittel als zusätzliche Komponente Wasser enthält, ist die Verwendung eines wasserlöslichen Katalysators in Form eines Alkalimetallpersulfats oder Ammoniumpersulfats bevorzugt.

Der Radikalstarter wird üblicherweise in einer Menge von 0.01 bis 5 Gew.-%, insbesondere von 0,05 bis 3 Gew.-%, bevorzugt 0,5 bis 1,5 Gew.-%, bezogen auf die gesamte Zusammensetzung, eingesetzt.

Zusätzlich zu dem Radikalstarter kann ein Co-Initiator eingesetzt werden, welcher oftmals auch als Beschleuniger bezeichnet wird. Dieser Co-Initiator ist insbesondere ein tertiäres Amin, ein Übergangsmetallsalz oder ein Übergangsmetallkomplex. Als Co-Initiator geeignete tertäre Amine sind insbesondere ausgewählt aus der Gruppe bestehend aus Di- oder Trialkanolaminen, bevorzugt Di- oder Triethanolamin oder deren Mischung, N,N-Dimethylanilin, N,N-Diethylanilin, N,N-Bis(hydroxyalkyl)aniline wie N,N-Bis(2-hydroxyethyl)anilin, N,N-Alkylhydroxyalkylaniline wie N-Ethyl-N-hydroxyethylanilin, N,N-Dimethyl-*p*-toluidin, N,N-Diethyl-*p*-toluidin, N-Methyl-N-hydroxyethyl-*p-*toluidin, N,N-Bis(2-hydroxyethyl)-*p*-toluidin sowie alkoxylierte N,N-Bis(hydroxyethyl)-*p*-toluidine, N-ethoxyliertes *p*-Toluidin, N,N-Bis(2-hydroxyethyl)-xylidin, N-Alkylmorpholin und Mischungen davon. Geeignete Übergangsmetallsalze und Übergangsmetallkomplexe sind beispielsweise Salze und Komplexe von Cobalt, Nickel, Kupfer, Mangan oder Vanadium.

Das Injektionsmittel kann darüber hinaus flüssige Additive, beispielsweise in Form von wässrigen Kunststoffdispersionen oder Polyethylenglykole enthalten. Unter wässrigen Kunststoffdispersionen werden im vorliegenden Dokument sowohl Kunststoffdispersionen verstanden, deren feste Kunststoffbestandteile bereits vor der Herstellung des Injektionsmittels in Wasser dispergiert vorliegen als auch feste, insbesondere pulverförmige, Kunststoffdispersionsanteile, welche erst bei der Verwendung des Injektionsmittels mit Wasser in Berührung kommen und darin dispergierbar sind. Je nach Ausführungsform des Injektionsmittels wird die wässrige Kunststoffdispersion als dispergierbarer Feststoff oder als bereits dispergierter Feststoff eingesetzt. Besonders geeignete wässrige Kunststoffdispersionen sind im Zusammenhang mit der vorliegenden Erfindung solche auf Basis von (Meth)acrylatpolymeren, Copolymeren aus (Meth)acrylaten und Styrol, Copolymeren aus Styrol und Butadien und Copolymeren aus Vinylacetat, Ethylen und gegebenenfalls einem Vinylester.

Zusätzlich oder alternativ kann dem Injektionsmittel ein Feinstzement zugesetzt werden, der eine Feinheit (bestimmt anhand des Siebrückstands) von maximal d₉₅ ≤ 16 µm, bevorzugt d₉₅ ≤ 10 µm und am meisten bevorzugt von d₉₅ ≤ 6 µm aufweist.

Zudem kann das Injektionsmittel Farbstoffe enthalten. Bevorzugt ist es, wenn das Injektionsmittel keine Viskositätssteigernden Bestandteile enthält, da dies die Funktion des Injektionsmittels beeinträchtigen kann. Besonders bevorzugt ist es, wenn das Injektionsmittel aus den vorgenannten Bestandteilen, d.h. dem Prepoylmer, gegebenenfalls Wasser, Radikalstarter, Co-Initiator, sowie gegebenenfalls wässrigen Kunststoffdispersionen oder Polyethylenglykolen und/oder Feinstzement, besteht.

Ein weiterer Aspekt der vorliegenden Erfindung betrifft ein Injektionsmittel, das ein vinylterminiertes Prepolymer, wie vorstehend beschrieben, enthält oder aus diesem besteht, zur Abdichtung beispielsweise von Bauwerken, Tunneln oder Minen, wobei das Injektionsmittel als weitere Bestandteile einen Katalysator und gegebenenfalls die im Vorstehenden für die entsprechende Verwendung beschriebenen Zusatzstoffe enthält.

Ein weiterer Aspekt der vorliegenden Erfindung betrifft die Verwendung eines vinylterminierten Prepolymers, wie vorstehend beschrieben, zur Herstellung von Superabsorbern, wobei das Prepolymer, gegebenenfalls mit weiteren Vinylmonomeren polymerisiert wird. Als weitere Vinylmonomere sind insbesondere (Meth)acrylate, bevorzugt bei neutralem pH-Wert (d.h. pH 7) ionische und insbesondere anionische (Meth)acrylate, und besonders bevorzugt Acrylsäure oder Methacrylsäure (diese liegen bei neutralem pH in deprotonierter Form vor), geeignet.

Ein weiterer Aspekt der vorliegenden Erfindung betrifft Superabsorber, die durch Polymerisation eines vinylterminierten Prepolymers, wie vorstehend beschrieben, gegebenenfalls unter Zusatz von weiteren Vinylmonomeren, gemäß den vorstehenden Angaben, erhältlich sind.

Ein weiterer Aspekt der vorliegenden Erfindung betrifft schließlich die Verwendung eines vinylterminierten Prepolymers, wie vorstehend beschrieben, zur Herstellung von Betonverflüssigern, wobei das Prepolymer, gegebenenfalls mit weiteren Monomeren, polymerisiert wird. Auch in diesem Fall handelt es sich bei den einzubeziehenden weiteren Monomeren zweckmäßig um polare, bevorzugt ionische und insbesondere anionische Monomere, die besonders bevorzugt Acrylsäure oder Methacrylsäure umfassen.

Ein letzter Aspekt der vorliegenden Erfindung betrifft schließlich einen Betonverflüssiger, der erhältlich ist durch Homopolymerisation eines vinylterminierten Prepolymers wie vorstehend beschrieben oder durch Copolymerisation dieser vinylterminierten Prepolymere mit anderen Vinylmonomeren. Im Rahmen dieses Aspekts sollten die Prepolymere im Mittel nur eine Vinylfunktion aufweisen. Bei einem höheren Anteil an Vinylfunktionen kann es zu einer Vernetzung der entstehenden Polymere kommen, was sich negativ auf die Eigenschaften des Polymers als Betonverflüssiger auswirkt. Die für die Herstellung des Betonverflüssigers einzubeziehenden Vinylmonomere entsprechen den im Vorstehenden für die Verwendung des vinylterminierten Prepolymers zur Herstellung von Betonverflüssigern genannten Vinylmonomeren.

Die Prepolymere weisen gegenüber den im Stand der Technik bekannten Prepolymeren auf Basis von Polyethern den Vorteil einer geringen Viskosität bei gleichzeitig hoher Wasserlöslichkeit auf. Werden diese Prepolymere zur Herstellung von Injektionsmitteln herangezogen, so lassen sich über das Verhältnis von Vinylverbindung zu Polyether gezielt die mechanische Festigkeit, die Bruchdehnung, das Quellverhalten und die Wasserlöslichkeit einstellen. Zudem enthalten die Prepolymere im Verhältnis zu ihrem Molekulargewicht nur wenige Esterbindungen, was eine verbesserte Stabilität gegenüber alkalischen Medien zur Folge hat.

Im Folgenden soll die vorliegende Erfindung anhand einiger Beispiele näher illustriert werden.

### Beispiele:

### Bestimmung der relevanten Eigenschaften

Die Viskositäten der Prepolymere sowie der 50%-igen wässrigen Lösungen der Prepolymere wurden bei 23°C mit einem Physica MCR101 Viskosimeter gemäß ISO 3219 mit einem koaxialen Zylindermesssystem (bei einem Kegelwinkel von 120°) bestimmt.

Das Aussehen der 50%-igen wässrigen Lösung wurde durch Augenschein bestimmt. Die Beobachtung "klar" indiziert, dass das Prepolymer gelöst war.

Die Gelzeit wurde für eine 50%-ige wässrige Lösung der Prepolymere (25 g) unter Zusatz von 0,5 g einer 10%-igen Lösung von Natriumpersulfat in Wasser und 0,25 g Triethanolamin bestimmt. Die Gelzeit entspricht der Zeit, bis visuell erste Gelstrukturen in der Reaktionslösung zu erkennen sind.

### Verwendete Materialien:

| | |
|---|---|
| Aduxol VP-6685 | Polyethylen oxid/Polypropylenoxid Blockcopolymer mit einem Molekulargewicht Mw von 2258 und einem Ethylenoxidanteil von 41 %, das Ethylenoxid bildet die Endblöcke (Schärer & Schläpfer AG) |
| Aduxol VP-11115 | Polyethylen oxid/Polypropylenoxid Blockcopolymer mit einem Molekulargewicht Mw von 1700 und einem Ethylenoxidanteil von 50 %, das Ethylenoxid bildet den mittleren Block (Schärer & Schläpfer AG) |
| Aduxol VP-11122 | mono-Methoxy-terminiertes Polyethylen oxid/Polypropylenoxid Blockcopolymer mit einem Molekulargewicht Mw von 500 und einem Ethylenoxidanteil von 50 %, das Polyethylenoxid ist Methoxy modifiziert (Schärer & Schläpfer AG) |
| Aduxol VP-11121 | mono-Methoxy-terminiertes Polyethylen oxid/Polypropylenoxid Blockcopolymer mit einem Molekulargewicht Mw von 1000 und einem Ethylenoxidanteil von 50 %, das Polyethylenoxid ist Methoxy modifiziert (Schärer & Schläpfer AG) |
| Aduxol VP-11132 | mono-Methoxy-terminiertes Polyethylen oxid/Polypropylenoxid Blockcopolymer mit einem Molekulargewicht Mw von 500 und einem Ethylenoxidanteil von 50 %, das Polypropylenoxid ist Methoxy modifiziert (Schärer & Schläpfer AG) |
| Aduxol VP-11128 | mono-Methoxy-terminiertes Polyethylen oxid/Polypropylenoxid Blockcopolymer mit einem Molekulargewicht Mw von 1000 und einem Ethylenoxidanteil von 50 %, das Polypropylenoxid ist Methoxy modifiziert (Schärer & Schläpfer AG) |
| MPEG500 | mono-Methoxy-terminiertes Polyethylenglycol mit einem Molekulargewicht Mw von 500 (BASF) |
| Desmodur T80P | Gemisch von 2,4- und 2,6-Toluylendiisocyanat im Verhältnis 80:20 (Bayer) |
| Vestanat IPDI | Isophorondiisocyanat (Evonik) |
| Desmodur N3300 | Hexamethylendiisocyanat-Trimerisat (Bayer) |
| 2-*t*BAEMA | 2-(tert-Butylamino)ethyl methacrylat (BASF) |
| DBTL | Dibutylzinndilaurat, Katalysator (Azelis) |
| OH-TEMPO | 4-hydroxy-2,2,6,6-tetramethylpiperidin-1-oxyl, Radikalfänger (Evonik) |

### Vorversuche

In Vorversuchen wurden zunächst Prepolymere auf Basis von Isocyanaten und Polyetherdiolen hergestellt (V-1 bis V-3). Hierzu wurden die Polyetherdiole in einer ersten Stufe mit Diisocyanaten zu einem Zwischenprodukt umgesetzt. In einer zweiten Stufe wurde dann Hydroxyethylmethacrylat (HEMA) zugegeben, um die noch vorhandenen Isocyanatgruppen mit Vinylfunktionalitäten zu modifizieren.

Bei den Versuchen V-1 und V-3 zeigte sich jedoch bereits nach der ersten Stufe eine vollständige Polymerisation des Systems. Bei Verwendung eines aliphatischen Diisocyanats (V-2) an Stelle eines aromatischen Diisocyanats wurde nach der zweiten Stufe ein Produkt erhalten, dass eine Viskosität von etwa 50000 mPa·s aufwies.

In einem zweiten Versuchsansatz wurde ein trifunktionelles Isocyanat (Desmodur N3300) zunächst mit Hydroxyethylmethacrylat und, in V-4, zusätzlich mit MPEG 500 umgesetzt. Das erhaltene Zwischenprodukt wurde in einem zweiten Schritt mit einem dihydroxyfunktionellen Polyether (Aduxol VP-6685) zum Endprodukt umgesetzt. Bei den Versuchen V-4 bis V-6 zeigte sich jedoch für die erhaltenen Produkte eine sehr hohe Viskosität von etwa 100.000 mPa·s und mehr, was auf einen signifikanten Anteil an Kettenverlängerung hindeutet.

Die Ergebnisse dieser Voruntersuchungen sowie die Zusammensetzungen der Produkte sind in der folgenden Tabelle 1 wiedergegeben.

**Tabelle 1**

| | | **V-1** | **V-2** | **V-3** | **V-4** | **V-5** | **V-6** |
|---|---|---|---|---|---|---|---|
| **1. Stufe** | | | | | | | |
| | Aduxol VP-6685 | 100 | 100 | | | | |
| | Aduxol VP-11115 | | | 100 | | | |
| | MPEG 500 | | | | 62 | | |
| | Desmodur T80P | 17,5 | | 22,5 | | | |
| | Vestanat IPDI | | 22,1 | | | | |
| | Desmodur N3300 | | | | 59,4 | 57,8 | 122 |
| | HEMA | | | | 9,8 | 13 | 55 |
| | Ethanol | | | | | 4,6 | |
| | DBTL | | 0,005 | | 0,01 | 0,01 | 0,01 |
| | OH-Tempo | | | | 0,02 | 0,02 | 0,02 |

| **2. Stufe** | | | | | | | |
|---|---|---|---|---|---|---|---|
| | DBTL | 0,005 | | | | | |
| | OH-TEMPO | 0,02 | 0,02 | 0,02 | | | |
| | HEMA | 13 | 12,9 | 16,7 | | | |
| | Aduxol VP-6685 | | | | 94 | 94 | 185 |

| **Eigenschaften** | | | | | | | |
|---|---|---|---|---|---|---|---|
| Viskosität des Prepolymers [mPa·s] | | Reaktionsmischung in 1. Stufe polymerisiert | 50000 | Reaktionsmischung in 1. Stufe polymerisiert | 99000 | 250000 | 340000 |
| Aussehen der 50%igen wässrigen Lsg | | - | klar | - | klar | klar | klar |
| Viskosität als 50%ige wässrige Lsg [mPa·s] | | - | 1200 | - | 3740 | 38000 | 15000 |
| Gel-Zeit [min] | | - | 40 | - | 29 | 12 | 7 |

### Beispiel 1 (erfindungsgemäß)

Die Zusammensetzungen 1 bis 8 wurden hergestellt, indem in einer Eintopfreaktion (Zusammensetzungen 1 bis 7) oder in einer zweistufigen Reaktion verschiedene einseitig mit Methoxygruppen funktionalisierte Polyether mit einer Hydroxyfunktion mit einem trifunktionellen Isocyanat (Desmodur N3300) und einem hydroxyfunktionellen Acrylat (Hydroxyethylmethacrylat oder Hydroxypropylmethacrylat (HPMA); Zusammensetzungen 1 bis 7) oder einem aminfunktionellen Acrylat (2-(*tert-*Butylamino)ethylmethacrylat; Zusammensetzung 8) umgesetzt wurden. Alle Produkte zeigten Viskositäten im Bereich von etwa 10.000 bis 50.000 mPa·s und waren gut verarbeitbar. Die Gelzeit der erhaltenen Zusammensetzungen lag im Bereich von 7 bis 30 min. Die genauen Zusammensetzungen sowie deren bestimmte Eigenschaften sind in Tabelle 2 wiedergegeben.

**Tabelle 2**

| | | **1** | **2** | **3** | **4** | **5** | **6** | **7** | **8** |
|---|---|---|---|---|---|---|---|---|---|
| **1. Stufe** | | | | | | | | | |
| | Aduxol VP-11122 | 75 | | | | | | | |
| | Aduxol VP-11121 | | 143 | | | | | | |
| | Aduxol VP-11132 | | | 75 | | | | | |
| | Aduxol VP-11128 | | | | 143 | | | | |
| | MPEG500 | | | | | 60 | 75 | 75 | 75 |
| | Demodur N3300 | 61 | 61 | 61 | 61 | 61 | 61 | 61 | 61 |
| | HEMA | 20,6 | 20,6 | 20,6 | 20,6 | 24,7 | 20,6 | | |
| | HPMA | | | | | | | 22,8 | |
| | DBTL | 0,01 | 0,01 | 0,01 | 0,01 | 0,01 | 0,01 | 0,01 | 0,01 |
| | OH-Tempo | 0,02 | 0,02 | 0,02 | 0,02 | 0,02 | 0,02 | 0,02 | 0,02 |

| **2. Stufe** | | | | | | | | | |
|---|---|---|---|---|---|---|---|---|---|
| 2-*t*BAEMA | | | | | | | | | 29,3 |

| **Eigenschaften** | | | | | | | | | |
|---|---|---|---|---|---|---|---|---|---|
| Viskosität des Prepolymers [mPa·s] | | 18000 | 25000 | 50000 | 40000 | 23000 | 9250 | 9550 | 13600 |
| Aussehen der 50%igen wässrigen Lsg | | klar | klar | klar | klar | klar | klar | klar | klar |
| Viskosität als 50% ige wässrige Lsg [mPa·s] | | 4980 | 13500 | 2035 | 1720 | 9100 | 2175 | 2450 | 6700 |
| Gel-Zeit | | 7 | 10 | 11 | 30 | 30 | 24 | 22 | 10 |

### Beispiel 2

In weiteren Untersuchungen sollte geprüft werden, wie sich ein Zusatz von difunktionellen Isocyanaten auf die erhaltenen Prepolmere auswirkt. Als Referenzbeispiel dieser Untersuchungen diente die Zusammensetzung 5, die nur trifunktionelles Isocyanat enthält. In den Zusammensetzungen 9, 10 und 11 wurde die mittlere NCO-Funktionalität auf 2,75 bzw. 2,5 vermindert. In der Vergleichszusammensetzung 7 wurde die mittlere NCO-Funktionalität weiter auf 2,24 vermindert. Für diese Zusammensetzung zeigte sich jedoch eine vollständige Polymerisation, so dass das erhaltene Produkt nicht mehr verwertbar war. Die Zusammensetzungen 9 bis 11 waren auch nach 2 Monaten Lagerung bei Raumtemperatur noch flüssig, während sich das Prepolymer gemäß Zusammensetzung 5 bereits nach 2 Wochen verfestigte. Weiterhin konnte bei den 50%-igen Lösungen der Zusammensetzungen 9 bis 11 keine Luft (in Form von Blasen) in der Lösung beobachtet werden, was bei der Referenzzusammensetzung 5 der Fall war.

Die genauen Zusammensetzungen der untersuchten Proben sowie deren bestimmte Eigenschaften sind in der folgenden Tabelle 3 wiedergegeben.

In einer weiteren Versuchsreihe wurde untersucht, wie sich unterschiedliche Verhältnisse von MPEG 500 und HEMA auf das entstehende Polymer auswirken. Als Referenz für diese Untersuchungen wurde Beispiel 6 verwendet, in dem das Verhältnis von MPEG 500 zu HEMA 1:1 betrug. In den Beispielen 12 und 13 wurde dieses Verhältnis auf 1:2 beziehungsweise 2:1 eingestellt. Dabei zeigte sich, dass die Veränderung des Verhältnisses einen nur geringfügigen Einfluss auf die Eigenschaften des entstehenden Prepolymers hatte. In allen Fällen wurden gut verarbeitbare Produkte erhalten.

**Tabelle 3**

| | | **5** | **9** | **10** | **11** | **V-7** | **6** | **12** | **13** |
|---|---|---|---|---|---|---|---|---|---|
| **1. Stufe** | | | | | | | | | |
| | MPEG500 | 60 | 60 | 60 | 50 | 60 | 75 | 100 | 100 |
| | Desmodur N3300 | 61 | 45,8 | 30,5 | 45,8 | 15,3 | 61 | 122 | 61 |
| | IPDI | | 8,8 | 17,6 | 8,8 | 26,4 | | | |
| | HEMA | 24,7 | 24,7 | 24,7 | 27,5 | 24,7 | 20,6 | 55 | 13,7 |
| | DBTL | 0,01 | 0,01 | 0,01 | 0,01 | 0,01 | 0,01 | 0,01 | 0,01 |
| | OH-Tempo | 0,02 | 0,02 | 0,02 | 0,02 | 0,02 | 0,02 | 0,02 | 0,02 |
| Ø-NCO-Funktionalität | | 3 | 2,75 | 2,5 | 2,75 | 2,24 | 3 | 3 | 3 |

| **Eigenschaften** | | | | | | | | | |
|---|---|---|---|---|---|---|---|---|---|
| Viskosität des Prepolymers [mPa·s] | | 23000 | 18000 | 14000 | 14000 | polymerisiert | 9250 | 31000 | 7600 |
| Aussehen der 50%igen wässrigen Lsg | | klar | klar | milchig | opak | - | klar | klar | klar |
| Viskosität als 50%ige wässrige Lsg [mPa·s] | | 9100 | 1400 | 1000 | 1350 | - | 2175 | n.b. | 3450 |
| Gel-Zeit [Min] | | 30 | 20 | 25 | 12 | - | 24 | 15 | 22 |

| | | | | | | | | | |
|---|---|---|---|---|---|---|---|---|---|
| n.b. = nicht bestimmt. | | | | | | | | | |

### Beispiel 3

Im Rahmen dieses Beispiels sollte untersucht werden, inwieweit die Herstellung der vinylterminierten Prepolymere im Rahmen eines sequentiellen oder Eintopfverfahrens Auswirkungen auf die erhaltenen Eigenschaften der Prepolymere hat. Dazu wurde in der Zusammensetzung 14 zunächst MPEG 500 mit einem trifunktionelle Isocyanat (Desmodur N3300) umgesetzt und das erhaltene Produkt in einer zweiten Stufe mit Hydroxyethylmethacrylat zum Endprodukt umgesetzt. In der Zusammensetzung 15 wurde das trifunktionelle Isocyanat zunächst mit Hydroxyethylmethacrylat umgesetzt und das erhaltene Produkt anschließend mit MPEG 500 zum Endprodukt umgesetzt. Bei der Zusammensetzung 12 wurden alle Komponenten in einem Eintopfverfahren miteinander umgesetzt. Dabei zeigte sich, dass die exakte Durchführung des Verfahrens einen nur geringen Einfluss auf die Viskosität des erhaltenen Prepolymers hatte (sh. Tabelle 4). Da das Eintopfverfahren mit dem geringsten zeitlichen Aufwand verbunden ist, ist dieses Verfahren für die Herstellung der vinylterminierten Prepolymere bevorzugt.

**Tabelle 4**

| | | **12** | **14** | **15** |
|---|---|---|---|---|
| **1. Stufe** | | | | |
| | MPEG500 | 100 | 100 | |
| | Desmodur N3300 | 122 | 122 | 122 |
| | HEMA | 55 | | 55 |
| | DBTL | 0,01 | 0,01 | 0,01 |
| | OH-Tempo | 0,02 | 0,02 | 0,02 |

| **2. Stufe** | | | | |
|---|---|---|---|---|
| | HEMA | | 55 | |
| | MPEG500 | | | 100 |

| **Eigenschaften** | | | | |
|---|---|---|---|---|
| Viskosität des Prepolymers [mPa·s] | | 31000 | 30000 | 14000 |

## Patentansprüche

1. Verwendung eines vinylterminierten Prepolymers auf Basis von Polyethern als Injektionsmittel oder Bestandteil eines Injektionsmittel zur Abdichtung von Bauwerken, Tunneln oder Minen,
wobei das vinylterminierte Prepolymer, erhältlich ist durch Umsetzung
i) eines Polyethers mit genau einer gegenüber Isocyanaten reaktiven funktionellen Gruppe mit
ii) mindestens einem Polyisocyanat, das eine mittlere Isocyanatfunktionalität im Bereich von 2,4 bis 3,5 aufweist, und
iii) mindestens einer Vinylverbindung, die genau eine gegenüber Isocyanaten reaktive funktionelle Gruppen aufweist,
wobei das Molverhältnis von Polyether i) zu Vinylverbindung iii) im Bereich von 3:1 bis 1:3 liegt und das Verhältnis der Summe der Molmengen von Polyether und Vinylverbindung zu Isocyanatgruppen ii) im Bereich von 1,5:1 bis 1:1,5 liegt, und
wobei das vinylterminierte Prepolymer mit einem Radikalstarter und ggf. weiteren Komponenten in einen Hohlraum eingespritzt wird, **dadurch gekennzeichnet, dass** der Anteil des Polyethers im vinylterminierten Prepolymer mindestens 30 Gew.-% und bevorzugt mindestens 33 Gew.-%, bezogen auf das Gesamtgewicht des Prepolymers, ausmacht.

2. Verwendung nach Anspruch 1, wobei das Prepolymer weniger als 10 Gew.-% und bevorzugt weniger als 5 Gew.-% an Reaktionsprodukten aus Polyoxyalkylenpolyolen auf Basis von Startermolekülen mit drei oder mehr Hydroxyfunktionalitäten, in denen die Hydroxyfunktionalitäten ganz oder teilweise mit Methacrylsäure umgesetzt wurden, enthält.

3. Verwendung nach einem der Ansprüche 1 bis 2, wobei der Polyether mit einer gegenüber Isocyanaten reaktiven funktionellen Gruppe ein lineares Polyethylenglycol, Polypropylenglycol oder ein gemischtes Polyethylenglycol/Polypropylenglycol-co- oder Blockcopolymer, bevorzugt ein Polyethylenglycol, ist, das auf einer Seite mit einer Alkoxygruppe, vorzugsweise einer Methoxygruppe, modifiziert ist.

4. Verwendung nach einem der Ansprüche 1 bis 3, wobei der Polyether mit einer gegenüber Isocyanaten reaktiven funktionellen Gruppe ein Molekulargewicht Mw im Bereich von 200 bis 5000 g/mol, bevorzugt 300 bis 2000 g/mol aufweist.

5. Verwendung nach einem der Ansprüche 1 bis 4, wobei die mindestens eine Vinylverbindung ausgewählt ist aus Alkoholen, bevorzugt in Form von Allylalkohol, Hydroxyethyl(meth)acrylat und Hydroxypropyl(meth)acrylat, und Aminen, bevorzugt in Form von 2-(tert-Butylamino)ethyl methacrylat.

6. Verwendung nach einem der vorangehenden Ansprüche, wobei das mindestens eine Polyisocyanat ein Triisocyanat, bevorzugt ein aliphatisches Triisocyanat, und besonders bevorzugt ein Trimer von Hexamethylendiisocyanat, umfasst.

7. Verwendung nach Anspruch 6, wobei das mindestens eine Polyisocyanat zusätzlich ein aliphatisches Diisocyanat, bevorzugt in Form von Isophorondiisocyanat umfasst.

8. Verwendung nach einem der vorangehenden Ansprüche, wobei für die Umsetzung zusätzlich iv) eine aromatische oder aliphatische Verbindung, die weder als Polyether i) noch als Vinylverbindung iii) zu qualifizieren ist, einbezogen wird, die eine gegenüber Isocyanaten reaktive funktionelle Gruppe aufweist.

9. Verfahren zur Herstellung eines Injektionsmittels umfassend die Herstellung eines vinylterminierten Prepolymers wie in einem der Ansprüche 1 bis 8 angegeben, umfassend
i) die Zugabe mindestens eines Polyisocyanats mit einer mittleren Isocyanatfunktionalität im Bereich von 2,4 bis 3,5 entweder zu einem Polyether mit genau einer gegenüber Isocyanaten reaktiven funktionellen Gruppe oder zu mindestens einer Vinylverbindung, die genau eine gegenüber Isocyanaten reaktive funktionelle Gruppen aufweist,
ii) das Abreagierenlassen der Isocyanatgruppen mit den gegenüber Isocyanaten reaktiven funktionellen Gruppen,
iii) abhängig von dem im Schritt i) vorgelegten Bestandteil, die Zugabe des anderen Bestandteils, zum Reaktionsprodukt aus Polyisocyanat und dem vorgelegten Bestandteil, und
iv) das Abreagierenlassen des Reaktionsprodukts aus Polyisocyanat und dem vorgelegten Bestandteil mit dem anderen Bestandteil,
wobei der andere Bestandteil die mindestens eine Vinylverbindung, die eine gegenüber Isocyanaten reaktive funktionelle Gruppe aufweist, ist wenn der im Schritt i) vorgelegte Bestandteil der Polyether mit einer gegenüber Isocyanaten reaktiven funktionellen Gruppe ist, und umgekehrt, und
die Zugabe eines Radikalstarters und ggf. weiterer Komponenten.

10. Verfahren zur Herstellung eines Injektionsmittels umfassend die Herstellung eines vinylterminierten Prepolymers wie in nach einem der Ansprüche 1 bis 8 angegeben, umfassend
i) die Zugabe von mindestens einem Polyisocyanat mit einer mittleren Isocyanatfunktionalität im Bereich von 2,4 bis 3,5 zu einer Mischung aus einem Polyether mit genau einer gegenüber Isocyanaten reaktiven funktionellen Gruppe und mindestens einer Vinylverbindung, die genau eine gegenüber Isocyanaten reaktive funktionelle Gruppen aufweist,
ii) das Abreagierenlassen des Polyisocyanats mit dem Polyether und der Vinylverbindung.

11. Verfahren nach Anspruch 9 oder 10, wobei für die Reaktion der Isocyanatgruppen mit den gegenüber Isocyanaten reaktiven funktionellen Gruppen ein Katalysator, bevorzugt in Form von Dibutylzinnlaurat, zugesetzt wird, der bevorzugt mit einem Anteil im Bereich von 0,001 bis 1 Gew.-%, bezogen auf das Gesamtgewicht der Reaktionsmischung, eingesetzt wird.

12. Verwendung eines vinylterminierten Prepolymers wie in einem der Ansprüche 1 bis 8 angegeben zur Herstellung von Superabsorbern, wobei das Prepolymer, gegebenenfalls mit weiteren Vinylmonomeren, polymerisiert wird.

13. Verwendung eines vinylterminierten Prepolymers wie in einem der Ansprüche 1 bis 8 angegeben zur Herstellung von Betonverflüssigern, wobei das Prepolymer, gegebenenfalls mit weiteren Monomeren, polymerisiert wird.

14. Injektionsmittel zum Abdichten von Bauwerken, Tunneln oder Minen, umfassend ein vinylterminiertes Prepolymer wie in einem der Ansprüche 1 bis 8 angegeben, einen Radikalstarter und gegebenenfalls einen Co-Initiator.

## Claims

1. Use of a vinyl-terminated prepolymer based on polyethers as injection medium or constituent of an injection medium for sealing of built structures, tunnels or mines,
wherein the vinyl-terminated prepolymer is obtainable by reaction of
i) a polyether having precisely one functional group reactive toward isocyanates with
ii) at least one polyisocyanate having a mean isocyanate functionality in the range from 2.4 to 3.5, and
iii) at least one vinyl compound having precisely one functional group reactive toward isocyanates,
wherein the molar ratio of polyether i) to vinyl compound iii) is in the range from 3:1 to 1:3 and the ratio of the sum total of the molar amounts of polyether and vinyl compound to isocyanate groups ii) is in the range from 1.5:1 to 1:1.5, and
wherein the vinyl-terminated prepolymer is injected into a cavity together with a free-radical initiator and optionally further components, **characterized in that** the proportion of the polyether in the vinyl-terminated prepolymer amounts to at least 30% by weight and preferably at least 33% by weight, based on the total weight of the prepolymer.

2. Use according to Claim 1, wherein the prepolymer contains less than 10% by weight and preferably less than 5% by weight of reaction products formed from polyoxyalkylenepolyols based on starter molecules having three or more hydroxyl functionalities, in which all or some of the hydroxyl functionalities have been reacted with methacrylic acid.

3. Use according to any of Claims 1 to 2, wherein the polyether having a functional group reactive toward isocyanates is a linear polyethylene glycol, polypropylene glycol or a mixed polyethylene glycol/polypropylene glycol copolymer or block copolymer, preferably a polyethylene glycol modified at one end with an alkoxy group, preferably a methoxy group.

4. Use according to any of Claims 1 to 3, wherein the polyether having a functional group reactive toward isocyanates has a molecular weight Mw in the range from 200 to 5000 g/mol, preferably 300 to 2000 g/mol.

5. Use according to any of Claims 1 to 4, wherein the at least one vinyl compound is selected from alcohols, preferably in the form of allyl alcohol, hydroxyethyl (meth)acrylate and hydroxypropyl (meth)acrylate, and amines, preferably in the form of 2-(tert-butylamino)ethyl methacrylate.

6. Use according to any of the preceding claims, wherein the at least one polyisocyanate comprises a triisocyanate, preferably an aliphatic triisocyanate, and more preferably a trimer of hexamethylene diisocyanate.

7. Use according to Claim 6, wherein the at least one polyisocyanate additionally comprises an aliphatic diisocyanate, preferably in the form of isophorone diisocyanate.

8. Use according to any of the preceding claims, wherein the reaction additionally includes iv) an aromatic or aliphatic compound which cannot be qualified either as a polyether i) or as a vinyl compound iii), having a functional group reactive toward isocyanates.

9. Process for producing an injection medium, comprising the preparation of a vinyl-terminated prepolymer as specified in any of Claims 1 to 8, comprising
i) the addition of at least one polyisocyanate having a mean isocyanate functionality in the range from 2.4 to 3.5 either to a polyether having precisely one functional group reactive toward isocyanates or to at least one vinyl compound having precisely one functional group reactive toward isocyanates,
ii) allowing the isocyanate groups to react with the functional groups reactive toward isocyanates,
iii) depending on the constituent initially charged in step i), the addition of the other constituent to the reaction product formed from polyisocyanate and the initially charged constituent, and
iv) allowing the reaction product formed from polyisocyanate and the initially charged constituent to react with the other constituent,
wherein the other constituent is the at least one vinyl compound having a functional group reactive toward isocyanates when the constituent initially charged in step i) is the polyether having a functional group reactive toward isocyanates, and vice versa, and
the addition of a free-radical initiator and optionally further components.

10. Process for producing an injection medium, comprising the preparation of a vinyl-terminated prepolymer as specified in any of Claims 1 to 8, comprising
i) the addition of at least one polyisocyanate having a mean isocyanate functionality in the range from 2.4 to 3.5 to a mixture of a polyether having precisely one functional group reactive toward isocyanates and at least one vinyl compound having precisely one functional group reactive toward isocyanates,
ii) allowing the polyisocyanate to react with the polyether and the vinyl compound.

11. Process according to Claim 9 or 10, wherein, for the reaction of the isocyanate groups with the functional groups reactive toward isocyanates, a catalyst is added, preferably in the form of dibutyltin laurate, which is preferably used in a proportion in the range from 0.001% to 1% by weight, based on the total weight of the reaction mixture.

12. Use of a vinyl-terminated prepolymer as specified in any of Claims 1 to 8 for production of superabsorbents, wherein the prepolymer is polymerized, optionally with further vinyl monomers.

13. Use of a vinyl-terminated prepolymer as specified in any of Claims 1 to 8 for production of concrete plasticizers, wherein the prepolymer is polymerized, optionally with further monomers.

14. Injection medium for sealing of built structures, tunnels or mines, comprising a vinyl-terminated prepolymer as specified in any of Claims 1 to 8, a free-radical initiator and optionally a co-initiator.

## Revendications

1. Utilisation d'un prépolymère terminé par vinyle à base de polyéthers en tant que produit d'injection ou en tant qu'ingrédient d'un produit d'injection pour l'étanchéification de bâtiments, de tunnels ou de mines,
le prépolymère terminé par vinyle pouvant être obtenu par transformation
i) d'un polyéther doté d'exactement un groupe fonctionnel réactif envers des isocyanates avec
ii) au moins un polyisocyanate qui présente une fonctionnalité moyenne d'isocyanate dans la plage de 2,4 à 3,5, et
iii) au moins un composé vinylique qui présente exactement un groupe fonctionnel réactif envers des isocyanates,
le rapport molaire de polyéther i) sur le composé vinylique iii) se situant dans la plage de 3:1 à 1:3 et le rapport de la somme des quantités molaires de polyéther et de composé vinylique sur les groupes isocyanate ii) se situant dans la plage de 1,5:1 à 1:1,5, et
le prépolymère terminé par vinyle étant projeté avec un amorceur radicalaire et éventuellement d'autres composants dans une cavité, **caractérisée en ce que** la proportion du polyéther dans le prépolymère terminé par vinyle représente au moins 30 % en poids et préférablement au moins 33 % en poids, par rapport au poids total du prépolymère.

2. Utilisation selon la revendication 1, le prépolymère contenant moins de 10 % en poids et préférablement moins de 5 % en poids de produits de réaction de polyoxyalkylènepolyols à base de molécules d'amorceur dotées de trois fonctionnalités hydroxy ou plus, dans lesquels les fonctionnalités hydroxy ont été totalement ou partiellement transformées avec de l'acide méthacrylique.

3. Utilisation selon l'une quelconque des revendications 1 et 2, le polyéther doté d'un groupe fonctionnel réactif envers des isocyanates étant un polyéthylène glycol linéaire, un polypropylèneglycol ou un mélange polyéthylène glycol/polypropylèneglycol-copolymère ou polyéthylène glycol/polypropylèneglycol-copolymère à blocs, préférablement un polyéthylèneglycol, qui est modifié latéralement par un groupe alcoxy, de préférence un groupe méthoxy.

4. Utilisation selon l'une quelconque des revendications 1 à 3, le polyéther doté d'un groupe fonctionnel réactif envers des isocyanates présentant un poids moléculaire Mw dans la plage de 200 à 5 000 g/mole, préférablement 300 à 2 000 g/mole.

5. Utilisation selon l'une quelconque des revendications 1 à 4, l'au moins un composé vinylique étant choisi parmi des alcools, préférablement sous la forme d'alcool allylique, d'un (méth)acrylate d'hydroxyéthyle et d'un (méth)acrylate d'hydroxypropyle, et des amines, préférablement sous forme de méthacrylate de 2-(tert-butylamino) éthyle.

6. Utilisation selon l'une quelconque des revendications précédentes, l'au moins un polyisocyanate comprenant un triisocyanate, préférablement un triisocyanate aliphatique, et particulièrement préférablement un trimère de diisocyanate d'hexamethylène.

7. Utilisation selon la revendication 6, l'au moins un polyisocyanate comprenant de plus un diisocyanate aliphatique, préférablement sous forme de diisocyanate d'isophorone.

8. Utilisation selon l'une quelconque des revendications précédentes, dans laquelle, pour la transformation, iv) un composé aromatique ou aliphatique étant de plus inclus, qui n'est à qualifier ni de polyéther i), ni de composé vinylique iii), qui présente un groupe fonctionnel réactif envers des isocyanates.

9. Procédé pour la préparation d'un produit d'injection comprenant la préparation d'un prépolymère terminé par vinyle comme indiqué dans l'une quelconque des revendications 1 à 8, comprenant
i) l'ajout d'au moins un polyisocyanate doté d'une fonctionnalité moyenne d'isocyanate dans la plage de 2,4 à 3,5, soit à un polyéther doté d'exactement un groupe fonctionnel réactif envers des isocyanates, soit à au moins un composé vinylique qui présente exactement un groupe fonctionnel réactif envers des isocyanates,
ii) le fait de laisser réagir les groupes isocyanate avec les groupes fonctionnels réactifs envers des isocyanates,
iii) en fonction de l'ingrédient fourni dans l'étape i), l'ajout de l'autre ingrédient, au produit de réaction du polyisocyanate et de l'ingrédient fourni, et
iv) le fait de laisser réagir le produit de réaction du polyisocyanate et de l'ingrédient fourni avec l'autre ingrédient,
l'autre ingrédient étant l'au moins un composé vinylique qui présente un groupe fonctionnel réactif envers des isocyanates, lorsque l'ingrédient fourni dans l'étape i) est le polyéther doté d'un groupe fonctionnel réactif envers des isocyanates, et inversement, et
l'ajout d'un amorceur radicalaire et éventuellement d'autres composants.

10. Procédé pour la préparation d'un produit d'injection comprenant la préparation d'un prépolymère terminé par vinyle comme indiqué dans l'une quelconque des revendications 1 à 8, comprenant
i) l'ajout d'au moins un polyisocyanate doté d'une fonctionnalité moyenne d'isocyanate dans la plage de 2,4 à 3,5 à un mélange d'un polyéther doté d'exactement un groupe fonctionnel réactif envers des isocyanates et d'au moins un composé vinylique qui présente exactement un groupe fonctionnel réactif envers des isocyanates,
ii) le fait de laisser réagir le polyisocyanate avec le polyéther et le composé vinylique.

11. Procédé selon la revendication 9 ou 10, dans lequel, pour la réaction des groupes isocyanate avec les groupes fonctionnels réactifs envers des isocyanates, un catalyseur est ajouté, préférablement sous forme de laurate de dibutylétain, qui est utilisé préférablement en une proportion dans la plage de 0,001 à 1 % en poids, par rapport au poids total du mélange réactionnel.

12. Utilisation d'un prépolymère terminé par vinyle comme indiqué dans l'une quelconque des revendications 1 à 8 pour la préparation de superabsorbants, le prépolymère étant polymérisé, éventuellement avec des monomères vinyliques supplémentaires.

13. Utilisation d'un prépolymère terminé par vinyle comme indiqué dans l'une quelconque des revendications 1 à 8 pour la préparation de fluidifiants pour béton, le prépolymère étant polymérisé, éventuellement avec des monomères supplémentaires.

14. Produit d'injection pour l'étanchéification de bâtiments, de tunnels ou de mines, comprenant un prépolymère terminé par vinyle comme indiqué dans l'une quelconque des revendications 1 à 8, un amorceur radicalaire et éventuellement un co-initiateur.
